# DEMANDE DE BREVET EUROPEEN

(11) **EP 3 744 311 A1**
(43) Date de publication de la demande: **02.12.2020**
(21) Numéro de dépôt: 20173146.0
(22) Date de dépôt: 05.10.2007
(51) Int. Cl.: A61K 8/97, A61Q 19/08, A61P 17/00, A61K 36/55, A61K 36/064, A61K 8/06, A61K 36/23, A61K 36/82, A61K 36/899, A61K 36/73, A61K 36/48, A61K 36/42, A61K 36/534, A61K 8/04, A61K 36/06, A61P 17/18, A61K 8/9728, A61K 8/9794, A61K 8/9789

(54) **UTILISATION D'UN PRINCIPE ACTIF ISSU DE LIN AGISSANT SUR LE DERME PAPILLAIRE, POUR SON ACTION ANTI-AGE**

(30) Priorité: 05.10.2006 FR 0654098
(62) Demande divisionnaire de: 07858517.1
(71) Demandeur: Société Industrielle Limousine d'Application Biologique Dite SILAB, 19130 Objat (FR)
(72) Inventeur: PAUFIQUE, Jean, 19130 OBJAT (FR)
(74) Mandataire: Aquinov

(57) **Abrégé**

L'objet de l'invention est l'utilisation d'au moins un principe actif issu de Lin capable d'agir sur le derme papillaire, pour la préparation d'une composition cosmétique et/ou dermopharmaceutique destinée à lutter contre le vieillissement cutané.

L'invention se rapporte également à toute composition cosmétique et/ou dermopharmaceutique à action anti-âge contenant au moins une substance capable d'agir sur le derme papillaire.

## Description

La présente invention concerne l'utilisation cosmétique d'un principe actif issu de Lin agissant sur des marqueurs spécifiques du derme papillaire, pour lutter contre le vieillissement cutané.

L'invention se rapporte également aux compositions anti-âges comprenant au moins un principe actif issu de Lin agissant sur des marqueurs spécifiques du derme papillaire, ainsi qu'à un procédé d'obtention d'un principe actif issu de Lin agissant sur le derme papillaire et au principe actif obtenu.

Dans nos sociétés, l'esthétisme et l'apparence prennent une place de plus en plus importante. Pour améliorer leur image, beaucoup de gens ont tendance à vouloir paraître jeunes le plus longtemps possible et cherchent à estomper les manifestations visibles du vieillissement de la peau.

C'est pourquoi l'objectif de la présente invention est de proposer des produits capables de lutter efficacement contre le vieillissement cutané, pour aider à rajeunir et à revitaliser la peau en considérant les aspects du vieillissement de la peau sur les différentes couches cutanées.

La peau est constituée de deux tissus étroitement reliés l'un à l'autre : l'épiderme situé en surface, et le derme, sous-jacent, au sein duquel on distingue le derme papillaire, jouxtée à la jonction dermo-épidermique, riche en fibroblastes, et le derme réticulaire.

Le derme constitue le support solide de la peau. Il est composé de fibroblastes et d'un réseau microfibrillaire, la matrice extracellulaire, riche en collagènes, en élastine, en glycoprotéines et en protéoglycanes qui assurent soutien, extensibilité et résistance.

Le derme papillaire tient son nom de sa surface en papilles qui forment des saillies alternant avec des prolongements épidermiques. Il a une structure fibreuse très fine, riche en cellules, en capillaires sanguins, en fibres nerveuses et corpuscules tactiles.

Le derme papillaire contient notamment des fibres oxytalan qui forment un réseau perpendiculaire qui semble ancré dans la membrane basale. Ces fibres jouent un rôle important dans la résistance mécanique de la peau. Une augmentation de la synthèse de ces fibres permet de conforter le réseau perpendiculaire et donc de consolider la résistance mécanique de la peau.

Le collagène XVI est exprimé à la fois par les kératinocytes et les fibroblastes du derme papillaire. Il contribue à l'intégrité structurelle de la zone du derme papillaire. Une augmentation de la synthèse de collagène XVI participe à l'amélioration de l'intégrité structurelle du derme papillaire.

Avec l'âge et les agressions extérieures, la matrice extracellulaire se désorganise et s'appauvrit en éléments constitutifs : la peau devient plus fine, plus fragile, plus sèche et perd de son élasticité, ce qui entraîne l'apparition de rides, la perte de luminosité du teint et le relâchement cutané.

Aussi, pour répondre à son objectif, la présente invention vise à agir à la fois sur l'épaisseur, l'élasticité et l'organisation structurelle du derme papillaire, en utilisant au moins un principe actif issu de Lin, capable d'agir sur la synthèse d'au moins une molécule fibreuse spécifique du derme papillaire, pour la préparation d'une composition cosmétique et/ou dermopharmaceutique destinée à lutter contre le vieillissement cutané.

En particulier, la présente invention se propose d'utiliser au moins un principe actif issu de Lin, capable d'agir sur la synthèse de molécules spécifiques du derme papillaire, notamment le collagène XVI et/ou les fibres oxytalan.

Le principe actif selon l'invention peut également agir sur la synthèse d'autres collagènes du derme papillaire : le collagène I, le collagène VI et le collagène XII, ainsi que sur la synthèse de fibrilline dans le derme papillaire.

L'utilisation selon la présente invention permet de lutter contre le vieillissement cutané en agissant sur le derme papillaire, en particulier :
- en augmentant la densité du derme papillaire, donc la densité de la peau, par stimulation de la synthèse du collagène XVI, et/ou
- en augmentant l'élasticité de la peau par stimulation de la synthèse des fibres d'oxytalan, fibre spécifique du derme papillaire jouant un rôle important dans la résistance mécanique et l'élasticité de la peau.

L'utilisation selon la présente invention peut également permettre :
- d'augmenter la densité du derme par synthèse des collagènes I,VI et XII, et/ou
- d'augmenter l'élasticité de la peau par stimulation de la synthèse de la fibrilline, molécule présente dans les fibres d'oxytalan.

Avantageusement, l'utilisation d'au moins un principe actif issu de Lin selon l'invention permet de lutter contre l'apparition des rides, la perte de luminosité du teint et/ou le relâchement cutané lié au vieillissement de la peau.

De manière préférentielle le principe actif issu de Lin capable d'agir sur le derme papillaire selon la présente invention est un principe actif obtenu par un procédé comprenant au moins une hydrolyse.

Le principe actif issu de Lin capable d'agir sur le derme papillaire selon l'invention est destiné à être incorporé au sein de compositions cosmétiques et/ou dermopharmaceutiques.

La composition cosmétique selon l'invention peut contenir de 0,01 à 20 % d'au moins un principe actif issu de Lin capable d'agir sur le derme papillaire.

L'administration d'une composition cosmétique contenant au moins principe actif issu de Lin selon l'invention, capable d'agir sur le derme papillaire, est de préférence effectuée par voie topique.

La composition selon l'invention peut se présenter sous forme de crèmes, émulsions huile-dans-eau, eau-dans-huile ou émulsions multiples, solutions, suspensions ou encore poudres. Selon un autre aspect, l'invention vise également un procédé d'obtention d'un principe actif issu de Lin capable d'agir sur le derme papillaire, ainsi que le principe actif obtenu.

La présente invention est maintenant décrite en détail en s'appuyant sur des résultats de tests.

### 1/ PROCEDE D'OBTENTION D'UN PRINCIPE ACTIF SELON L'INVENTION :

Le principe actif issu de Lin capable d'agir sur le derme papillaire, utile selon l'invention, peut être obtenu par un procédé comprenant au moins une hydrolyse.

Préférentiellement, il est obtenu par un procédé comprenant les étapes suivantes :
- solubilisation de Lin dans l'eau,
- hydrolyse(s) chimique et/ou enzymatique(s) successive(s) et/ou simultanée(s),
- séparation des phases soluble et insoluble,
- inactivation de ou des activité(s) enzymatique(s) résiduelle(s), de préférence par traitement thermique,
- purification de la fraction active par séparation décantation, filtration, centrifugation,
- concentration par filtration.

Des étapes supplémentaires peuvent également être ajoutées pour décolorer et/ou stabiliser le produit obtenu.

Préférentiellement, on entend par « Lin » des graines de lin de la variété *Linum usitatissimum,* réduites en poudre, sous forme de farine ou de semoule, ou des tourteaux de graines de lin de la variété *Linum usitatissimum* obtenus après extraction de la partie huileuse par pressage des graines.

### 2/ CARACTERISATION D'UN PRINCIPE ACTIF SELON L'INVENTION :

### 2-1/ Matières sèches :

On mesure le taux de matières sèches par passage à l'étuve à 105°C en présence de sable d'un échantillon de poids initial donné jusqu'à obtention d'un poids constant.

Le taux de matières sèches d'un principe actif issu de Lin selon l'invention est compris entre 5 et 200 g/l, plus particulièrement entre 15 et 60 g/l.

### 2-2/ Mesure du pH :

Le pH mesuré par la méthode potentiométrique à température ambiante conduit à des valeurs comprises entre 3 et 7, plus particulièrement entre 3 et 5.

### 2-3/ Détermination de la teneur en protéines

La teneur en protéines totales est déterminée par la méthode de LOWRY.

Le taux de protéines d'un principe actif issu de Lin selon l'invention est compris entre 1 et 65 g/l, plus particulièrement entre 3 à 20 g/l.

### 2-4/ Caractérisation de la fraction protéique

La caractérisation de la fraction protéique sont réalisées par FPLC (Fast Protein Liquid Chromatography).

Le chromatogramme montre qu'un principe actif issu de Lin selon l'invention est composé majoritairement de peptides de poids moléculaire inférieur à 5000 Daltons.

### 2-5/ Détermination de la teneur en sucres totaux

On utilise la méthode de DUBOIS. En présence d'acide sulfurique concentré et de phénol, les sucres réducteurs donnent un composé jaune orangé. A partir d'une gamme étalon, on peut déterminer le taux de sucres totaux d'un échantillon.

Le taux de sucres totaux d'un principe actif issu de Lin selon l'invention est compris entre 2 et 100g/l, préférentiellement entre 5 et 30g/l.

### 2-6/ Caractérisation des sucres monomères

La caractérisation des sucres monomères d'un principe actif issu de Lin selon l'invention est réalisée par chromatographie liquide haute performance (CLHP).

Les résultats obtenus montrent qu'un principe actif issu de Lin selon l'invention est composé, en ordre décroissant, de mannose, de glucose, de xylose et de rhamnose. En particulier sa fraction glucidique comprend au moins 50% de mannose.

### 2-7/ Détermination des acides uroniques

Les acides uroniques forment des dérivés furaniques, par cycloaddition sous l'action des acides minéraux forts. Les dérivés furaniques ainsi formés sont susceptibles de condenser avec différents réactifs en formant des complexes de coloration caractéristique : les chromophores, produit de la réaction, de couleur variable selon la nature de l'ose.

La réaction de l'acide galacturonique avec le Borax donne en présence du métahydroxydiphényl, une coloration rose permettant le dosage au spectrophotomètre à 520nm.

Cette analyse montre que le principe actif issu de Lin selon l'invention contient des acides uroniques.

### 2-8/ Caractérisation des carbohydrates

La détermination de la taille des carbohydrates d'un principe actif issu de Lin selon l'invention est réalisée par chromatographie liquide haute performance.

Le chromatogramme obtenu montre la présence d'environ 74% de monosaccharides de masse moléculaire inférieure à 180Da, d'environ 17% d'oligosaccharides de masse molaire comprise entre 180 et 2500Da et d'environ 9% de polysaccharides de masse molaire supérieure à 5000Da.

### 3. EVALUATION DE L'EFFET DU PRINCIPE ACTIF ISSU DE LIN SELON L'INVENTION

### 3.1 Evaluation de l'effet sur la densité du derme papillaire

### a - Evaluation in vitro de l'expression des ARNm codant le collagène I par des fibroblastes vieillis issus de dermes papillaires

L'objectif de cette étude est d'évaluer l'effet d'un principe actif issu de Lin selon l'invention sur l'expression des ARNm (Acides Ribonucléiques messagers) codant le collagène I sur des fibroblastes vieillis issus de dermes papillaires en comparaison avec des fibroblastes jeunes issus de dermes papillaires.

L'étude est réalisée par PCR (Amplification en Chaîne par Polymérase) selon le protocole opératoire ci-dessous.

A J1, les fibroblastes humains jeunes et vieillis sont ensemencés et incubés.

A J3, les cellules sont cultivées en présence d'un principe actif issu de Lin selon l'invention à 1%. On conserve également des cellules non traitées qui servent de témoin.

A J4, les cellules sont récupérées et les ARN (Acide Ribonucléique) totaux sont extraits. Les ARN sont reverse-transcripts et les ADN (Acide Désoxyribonucléique) complémentaires obtenus sont analysés par PCR quantitative.

Les résultats obtenus en pourcentage d'ARNm de collagène I exprimés, sont présentés dans le tableau suivant :

| | Taux d'ARNm de collagène I (%) | |
|---|---|---|
| | Fibroblastes humains jeunes | Fibroblastes humains vieillis |
| Témoin non traité | 100 | 49 |
| Principe actif issu de Lin 1% | | 96 |

On constate que les fibroblastes humains vieillis perdent 51% de leur capacité d'expression des ARNm codant le collagène I par rapport à des fibroblastes humains jeunes. Le principe actif issu de Lin selon l'invention permet de récupérer la capacité d'expression des ARNm codant le collagène I des fibroblastes vieillis issus de dermes papillaires.

### b - Evaluation in vitro de la synthèse de collagène I par des fibroblastes jeunes et des fibroblastes vieillis issus de dermes papillaires,

L'objectif de cette étude est d'évaluer l'effet d'un principe actif issu de Lin selon l'invention sur la synthèse de collagène I sur des fibroblastes jeunes et vieillis issus de dermes papillaires.

L'étude est réalisée par technique ELISA selon le protocole opératoire ci-dessous.

A J1, les fibroblastes humains jeunes et vieillis sont ensemencés et incubés.

A J2, les cellules sont cultivées en présence d'un principe actif issu de Lin selon l'invention à 1%. On conserve également des cellules non traitées qui servent de témoin.

A J3, les surnageants sont récupérés, puis dosés par technique ELISA. Le dosage ELISA est réalisé avec les moyens suivants :
- anticorps primaire : anticorps monoclonal de souris anti-collagène I
- anticorps secondaire : anticorps anti-IgG de souris
- système de révélation : peroxydase et substrat TMB

Les résultats obtenus en pourcentage de collagène I exprimés sont présentés dans le tableau suivant :

| | Taux de collagène I (%) |
|---|---|
| | Fibroblastes humains jeunes |
| Témoin non traité | 100 |
| Principe actif issu de Lin 1% | 223 |

On constate que le principe actif selon l'invention issu de Lin agit en augmentant la synthèse de collagène I dans des cultures de fibroblastes humains jeunes issus de dermes papillaires.

| | Taux de collagène I (%) |
|---|---|
| | Fibroblastes humains vieillis |
| Témoin non traité | 100 |
| Principe actif issu de Lin 1% | 151 |

On constate que le principe actif selon l'invention issu de Lin agit également en augmentant la synthèse de collagène I dans des cultures de fibroblastes humains vieillis issus de dermes papillaires.

### c- Evaluation in vitro de l'expression des ARNm codant le collagène VI par des fibroblastes vieillis issus de dermes papillaires

L'objectif de cette étude est d'évaluer l'effet d'un principe actif issu de Lin selon l'invention sur l'expression des ARNm codant le collagène VI sur des fibroblastes vieillis issus de dermes papillaires en comparaison avec des fibroblastes jeunes issus de dermes papillaires.

L'étude est réalisée par PCR (Amplification en Chaîne par Polymérase) selon le protocole opératoire ci-dessous.

A J1, les fibroblastes humains jeunes et vieillis sont ensemencés et incubés.

A J3, les cellules sont cultivées en présence d'un principe actif issu de Lin selon l'invention à 1%. On conserve également des cellules non traitées qui servent de témoin.

A J4, les cellules sont récupérées et les ARN (Acide Ribonucléique) totaux sont extraits. Les ARN sont reverse-transcripts et les ADN (Acide Désoxyribonucléique) complémentaires obtenus sont analysés par PCR quantitative.

Les résultats obtenus en pourcentage d'ARNm (ARN messagers) de collagène VI exprimés, sont présentés dans le tableau suivant :

| | Taux d'ARNm de collagène VI (%) | |
|---|---|---|
| | Fibroblastes humains jeunes | Fibroblastes humains vieillis |
| Témoin non traité | 100 | 40 |
| Principe actif issu de Lin 1% | | 87 |

On constate que les fibroblastes humains vieillis perdent 60% de leur capacité d'expression des ARNm de collagène VI. Le principe actif issu de Lin selon l'invention permet d'améliorer la capacité d'expression des ARNm codant le collagène VI des fibroblastes vieillis issus de dermes papillaires.

### d - Evaluation in vitro de l'expression des ARNm codant le de collagène XII par des fibroblastes vieillis issus de dermes papillaires

L'objectif de cette étude est d'évaluer l'effet d'un principe actif issu de Lin selon l'invention sur l'expression des ARNm codant le collagène XII sur des fibroblastes vieillis issus de dermes papillaires en comparaison avec des fibroblastes humains jeunes issus de dermes papillaires. L'étude est réalisée par PCR (Amplification en Chaîne par Polymérase) selon le protocole opératoire ci-dessous.

A J1, les fibroblastes humains jeunes et vieillis sont ensemencés et incubés.

A J3, les cellules sont cultivées en présence d'un principe actif issu de Lin selon l'invention à 1%. On conserve également des cellules non traitées qui servent de témoin.

A J4, les cellules sont récupérées et les ARN (Acide Ribonucléique) totaux sont extraits. Les ARN sont reverse-transcripts et les ADN (Acide Désoxyribonucléique) complémentaires obtenus sont analysés par PCR quantitative.

Les résultats obtenus en pourcentage d'ARNm (ARN messagers) de collagène XII exprimés, sont présentés dans le tableau suivant :

| | Taux d'ARNm de collagène XII (%) | |
|---|---|---|
| | Fibroblastes humains jeunes | Fibroblastes humains vieillis |
| Témoin non traité | 100 | 35 |
| Principe actif issu de Lin 1% | | 99 |

On constate que les fibroblastes humains vieillis perdent 65% de leur capacité d'expression des ARNm de collagène XII. Le principe actif issu de Lin selon l'invention permet de récupérer la capacité d'expression des ARNm codant le collagène XII des fibroblastes issus de dermes papillaires vieillis.

### e - Evaluation in vitro de l'expression des ARNm codant le collagène XVI par des fibroblastes vieillis issus de dermes papillaires

L'objectif de cette étude est d'évaluer l'effet d'un principe actif issu de Lin selon l'invention sur l'expression des ARNm codant le collagène XVI sur des fibroblastes vieillis issus de dermes papillaires en comparaison de fibroblastes humains jeunes issus de dermes papillaires. L'étude est réalisée par PCR (Amplification en Chaîne par Polymérase) selon le protocole opératoire ci-dessous.

A J1, les fibroblastes humains jeunes et vieillis sont ensemencés et incubés.

A J3, les cellules sont cultivées en présence d'un principe actif issu de Lin selon l'invention à 1%. On conserve également des cellules non traitées qui servent de témoin.

A J4, les cellules sont récupérées et les ARN (Acide Ribonucléique) totaux sont extraits. Les ARN sont reverse-transcripts et les ADN (Acide Désoxyribonucléique) complémentaires obtenus sont analysés par PCR quantitative.

Les résultats obtenus en pourcentage d'ARNm de collagène XVI exprimés, sont présentés dans le tableau suivant :

| | Taux d'ARNm de collagène XVI (%) | |
|---|---|---|
| | Fibroblastes humains jeunes | Fibroblastes humains vieillis |
| Témoin non traité | 100 | 43 |
| Principe actif issu de Lin 1% | | 120 |

On constate que les fibroblastes humains vieillis perdent 57% de leur capacité d'expression des ARNm de collagène XVI. Le principe actif issu de Lin selon l'invention permet de récupérer la capacité d'expression des ARNm codant le collagène XVI des fibroblastes issus de dermes papillaires vieillis.

### 3.2 Evaluation de l'effet sur l'élasticité du derme papillaire - Evaluation in vitro de l'effet sur les fibres oxytalan

La fibrilline I est une glycoprotéine essentielle dans la formation des fibres élastiques, présentes dans les fibres oxytalan.

L'objectif de cette étude est d'évaluer l'effet d'un principe actif issu de lin selon l'invention sur l'expression des ARNm codant la fibrilline I sur des fibroblastes vieillis issus de dermes papillaires en comparaison avec des fibroblastes humains jeunes issus de dermes papillaires. L'étude est réalisée par PCR (Amplification en Chaîne par Polymérase) selon le protocole opératoire ci-dessous.

A J1, les fibroblastes humains jeunes et vieillis sont ensemencés et incubés.

A J3, les cellules sont cultivées en présence d'un principe actif issu de Lin selon l'invention à 1%. On conserve également des cellules non traitées qui servent de témoin.

A J4, les cellules sont récupérées et les ARN (Acide Ribonucléique) totaux sont extraits. Les ARN sont reverse-transcripts et les ADN (Acide Désoxyribonucléique) complémentaires obtenus sont analysés par PCR quantitative.

Les résultats obtenus en pourcentage d'ARNm de fibrilline I exprimés, sont présentés dans le tableau suivant :
L'études est réalisée

| | Taux d'ARNm de fibrilline I (%) | |
|---|---|---|
| | Fibroblastes humains jeunes | Fibroblastes humains vieillis |
| Témoin non traité | 100 | 35 |
| Principe actif issu de Lin 1% | | 162 |

On constate que les fibroblastes humains vieillis perdent 65% de leur capacité à exprimer les ARNm de fibrilline I par rapport aux fibroblastes humains jeunes issus de dermes papillaires. Le principe actif issu de Lin selon l'invention permet d'améliorer la capacité d'expression des ARNm de fibrilline I sur des cultures de fibroblastes humains vieillis issus de dermes papillaires.

### 3.3 Evaluation de l'effet in vivo de l'effet raffermissant

L'objectif de cette étude est de montrer l'efficacité in vivo du principe actif issu de Lin selon l'invention formulé à 4% en émulsion contre placebo sur les propriétés biomécaniques de la peau, au niveau du bas du visage.

L'étude est réalisée sur 18 volontaires sains de sexe féminin.

Les mesures sont réalisées au niveau du bas du visage à l'aide d'un Cutomètre : la peau est aspirée par une sonde à dépression constante pendant une durée constante. La profondeur de pénétration de la peau dans la sonde est mesurée par deux prismes optiques, ce qui permet d'obtenir des courbes à partir desquelles il est possible de calculer divers paramètres caractéristiques des propriétés mécaniques de la peau. Parmi ces paramètres, la présente étude consiste à observer les paramètres suivants :
- X qui représente la tonicité de la peau (si X augmente, la tonicité augmente),
- R0 qui représente la fermeté de la peau, (si R0 augmente, la peau est plus ferme)
- R9 qui représente la fatigabilité de la peau, (si R9 diminue, « l'effet fatigue » de la peau est moins important), et
- R2 qui représente l'élasticité (si R2 augmente, l'élasticité de la peau augmente).

Le protocole de l'étude est exposé en suivant.

Entre J-15 et J0, les volontaires arrêtent d'appliquer tout produit cosmétique sur le visage.

A J0, on détermine deux zones cutanées symétriques au niveau du bas du visage et on mesure les propriétés biomécaniques de la peau sur ces deux zones.

Entre J0 et J27, les volontaires appliquent bi-quotidiennement le placebo et le produit contenant le principe actif issu de Lin selon l'invention et le placebo.

A J28, on effectue des mesures des propriétés biomécaniques de la peau sur les deux zones du visage étudiées.

Entre J28 et J55, les volontaires appliquent bi-quotidiennement le placebo et le produit contenant le principe actif issu de Lin selon l'invention.

A J56, on effectue des mesures des propriétés biomécaniques de la peau sur les deux zones du visage étudiées.

Les résultats obtenus sont présentés dans le tableau ci-dessous :

| | Variation par rapport au placebo (%) |
|---|---|
| -R0 | 10% |
| R2 | 8% |
| R9 | -16% |
| -X | 14% |

Le principe actif issu de Lin selon l'invention permet d'augmenter la fermeté cutanée de 10%, la tonicité cutanée de 14% et l'élasticité de 8%.

De plus, on constate que des applications biquotidiennes du principe actif issu de Lin selon l'invention pendant 56 jours permettent de diminuer la fatigue de la peau de 16% par rapport au placebo.

### 4. EXEMPLES DE COMPOSITION

La présente invention couvre aussi les compositions cosmétiques et/ou dermopharmaceutiques incluant au moins un principe actif issu de Lin capable d'agir sur le derme papillaire selon la présente invention dans différentes formes galéniques, adaptées à l'administration par voie topique cutanée.

Ces compositions peuvent se présenter notamment sous forme de crèmes, émulsions huile-dans-eau, émulsions eau-dans-huile, émulsions multiples, solutions, suspensions ou poudres. Elles peuvent être plus ou moins fluides et avoir l'aspect d'une crème, d'une lotion, d'un lait, d'un sérum, d'une pommade, d'un gel, d'une pâte ou d'une mousse, ou sous forme solide. Ces compositions contiennent entre 0,01 et 20% en poids de principe(s) actif(s) issu(s) de Lin capable(s) d'agir sur le derme papillaire selon la présente invention.

Les exemples de composition qui suivent sont obtenus par mélange des différents composants. Les quantités indiquées sont données en pourcentage de poids.

### 4.1 Exemple d'une composition anti-rides destinée aux femmes

La formulation est la suivante :

| | | |
|---|---|---|
| - Principe actif selon l'invention : | | 4,0% |
| - Isononyl isononanoate : | | 5,0% |
| - Cetearyl octonoate : | 5,0% | |
| - Cetyl alcool : | | 4,0% |
| - Arachidyl alcool/Behenyl Alcool/ | | |
| Arachidyl glucoside : | 3,0% | |
| - PEG-100 stearate glyceryl stearate : | 2,0% | |
| - Glycerol : | | 2,0% |
| - Glycol palmitate : | | 1,0% |
| - Myreth-3 myristate : | 1,0% | |
| - Shea butter : | 1,0% | |
| - Polyacrilamide/C13-C14 paraffine/ | | |
| Laureth7 : | | 0,75% |
| - Phenonip : | | 0,7% |
| - Eau: | | 70,55% |

### 4.2 Exemple d'une composition anti-rides destinée aux hommes

La formulation est la suivante :

| | | |
|---|---|---|
| - Principe actif selon l'invention : | | 4,0% |
| - Acide stéarique : | | 7,0% |
| - Myristate isopropyl : | 7,0% | |
| - Stearyl alcool / Ceteareth 20 : | | 3,0% |
| - Stearyl stearate : | | 1,0% |
| - Huile minérale/Dibutyl lauryl glutamide : | 1,0% | |
| - Phenoxyethanol / parbenes : | 0,7% | |
| - Triethanolamine : | | 0,3% |
| - Eau: | | 76% |

### 4.3 Exemple d'une composition raffermissante

La formulation est la suivante :

| | |
|---|---|
| - Principe actif selon l'invention : | 4,0% |
| - Isononyl isononanoate : | 5,0% |
| - Arachidyl alcool/Behenyl Alcool/ Arachidyl glucoside : | 3,0% |
| - Cetearyl alcool / Cetearyl glucoside : | 2,0% |
| - Polyacrylamide / C13-14 Isoparaffin | |
| Laureth-7: | 2,0% |
| - Phenonip : | 0,7% |
| - Eau: | 83,3% |

## Revendications

1. Utilisation d'au moins un principe actif issu de Lin agissant sur la synthèse d'au moins une molécule fibreuse spécifique du derme papillaire, pour la préparation d'une composition cosmétique et/ou dermopharmaceutique destinée à lutter contre le vieillissement cutané.

2. Utilisation d'au moins un principe actif issu de Lin pour la préparation d'une composition cosmétique et/ou dermopharmaceutique selon la revendication 1, **caractérisée en ce que** ledit principe actif est issu de graines de Lin de la variété *Linum usitatissimum,* réduites en poudre, sous forme de farine ou de semoule, ou est issu de tourteaux de graines de Lin de la variété *Linum usitatissimum* obtenus après extraction de la partie huileuse par pressage des graines.

3. Utilisation d'au moins un principe actif issu de Lin pour la préparation d'une composition cosmétique et/ou dermopharmaceutique selon la revendication 1 ou 2, **caractérisée en ce que** ledit principe actif agit sur la synthèse du collagène XVI et/ou des fibres oxytalan du derme papillaire.

4. Utilisation d'au moins un principe actif issu de Lin pour la préparation d'une composition cosmétique et/ou dermopharmaceutique selon l'une des précédentes revendications, **caractérisée en ce que** ledit principe actif agit également sur la synthèse par le derme papillaire, du collagène I et/ou du collagène VI et/ou du collagène XII.

5. Utilisation d'au moins un principe actif issu de Lin pour la préparation d'une composition cosmétique et/ou dermopharmaceutique selon l'une des précédentes revendications, **caractérisée en ce que** ledit principe actif agit également sur la synthèse de la fibrilline dans le derme papillaire.

6. Utilisation d'au moins un principe actif issu de Lin pour la préparation d'une composition cosmétique et/ou dermopharmaceutique selon l'une des précédentes revendications, **caractérisée en ce que** la composition cosmétique et/ou dermopharmaceutique est destinée à lutter contre l'apparition des rides et/ou la perte de luminosité du teint et/ou le relâchement cutané, lié(s) au vieillissement de la peau.

7. Utilisation d'au moins un principe actif issu de Lin pour la préparation d'une composition cosmétique et/ou dermopharmaceutique selon l'une des précédentes revendications, **caractérisée en ce que** la composition cosmétique et/ou dermopharmaceutique est destinée à augmenter la densité du derme papillaire.

8. Utilisation d'au moins un principe actif issu de Lin pour la préparation d'une composition cosmétique et/ou dermopharmaceutique selon l'une des précédentes revendications, **caractérisée** en ce la composition cosmétique et/ou dermopharmaceutique est destinée à augmenter l'élasticité de la peau.

9. Procédé d'obtention d'un principe actif issu de Lin agissant sur la synthèse d'au moins une molécule fibreuse spécifique du derme papillaire, adapté pour une utilisation selon l'une quelconque des précédentes revendications, **caractérisé en ce qu'**il comprend au moins une étape d'hydrolyse.

10. Procédé d'obtention selon la revendication 9, **caractérisé en ce qu'**il comprend au moins les étapes suivantes :
- solubilisation de Lin dans l'eau,
- hydrolyse(s) chimique(s) et/ou enzymatique(s) successive(s) et/ou simultanée(s),
- séparation des phases soluble et insoluble,
- inactivation de ou des activité(s) enzymatique(s) résiduelle(s), de préférence par traitement thermique,
- purification de la fraction active par séparation décantation, filtration, centrifugation,
- concentration par filtration.

11. Principe actif issu de Lin agissant sur la synthèse d'au moins une molécule fibreuse spécifique du derme papillaire, obtenu par la mise en œuvre du procédé selon la revendication 9 ou 10, **caractérisé par** :
- un taux de matières sèches compris entre 15 et 60 g/l,
- un pH compris entre 3 et 5,
- une teneur en protéines comprise entre 3 et 20 g/l,
- une teneur en sucres totaux comprise entre 5 et 30g/l,
- la présence d'acides uroniques, et
- la présence de carbohydrates sous forme de monosaccharides, d'oligosaccharides et de polysaccharides.

12. Principe actif issu de Lin selon la précédente revendication, **caractérisé en ce qu'**il est issu de graines de lin de la variété *Linum usitatissimum,* réduites en poudre, sous forme de farine ou de semoule, ou est issu de tourteaux de graines de lin de la variété *Linum usitatissimum* obtenus après extraction de la partie huileuse par pressage des graines.

13. Composition cosmétique et/ou dermopharmaceutique destinée à lutter contre le vieillissement cutané, adaptée pour l'utilisation selon l'une quelconque des revendications 1 à 8, **caractérisée en ce qu'**elle contient entre 0,01% et 20% d'au moins un principe actif issu de Lin agissant sur la synthèse d'au moins une molécule fibreuse spécifique du derme papillaire.

14. Composition cosmétique et/ou dermopharmaceutique selon la revendication 13, **caractérisée en ce qu'**elle se présente sous forme de crème, émulsion huile-dans-eau, émulsion eau-dans-huile, émulsions multiples, solution, suspension ou poudre.
